# EUROPEAN PATENT SPECIFICATION

(11) **EP 2 624 809 B1**
(45) Date of publication and mention of the grant of the patent: **31.12.2014**
(21) Application number: 11754880.0
(22) Date of filing: 12.09.2011
(51) Int. Cl.: A61K 8/362, A61K 8/86, A61Q 19/02, A61K 8/36

(54) **A SKIN LIGHTENING COMPOSITION**
ZUSAMMENSETZUNG ZUR HAUTAUFHELLUNG
COMPOSITION D'ÉCLAIRCISSEMENT DE LA PEAU

(30) Priority: 26.11.2010 EP 10192732; 04.10.2010 IN MU27532010
(43) Date of publication of application: 14.08.2013
(73) Proprietor: Unilever N.V., 3013 AL Rotterdam (NL); Unilever PLC, London, Greater London EC4Y 0DY (GB)
(72) Inventor: BANDYOPADHYAY, Punam, Bangalore 560 066 (IN); GAURAV, Kumar, 825301 Jharkhand (IN); JHA, Sujeetkumar, Dist-Thane 421503, Maharashtra (IN); VORA, Shilpa Atul, Bangalore 560 066 (IN)
(74) Representative: Corsten, Michael Allan
(86) International application number: PCT/EP2011/065755
(87) International publication number: WO 2012/045550

(56) References cited:
- WO-A1-99/37280
- JP-A- 6 048 935
- JP-A- 2009 221 179

## Description

### Filed of the invention

The invention relates to a skin lightening composition; especially to a cream that provides faster kinetics of skin lightening as compared to conventional compositions.

### Background of the invention

Highly pleasing skin appearance is one of the most desired expectations from personal care products from most consumers around the world. In tropical countries where consumers generally have dark skin, there is a desire to have lighter skin appearance. In consumers who live far from the tropical countries e.g. the Caucasian people who generally have lighter skin, there is a problem of freckles and hyper pigmentation when they are exposed to bright sunshine and therefore they desire an even tanned tone of their skin. Most consumers experience blemishes on their skin after exposure to sun, on healing of wounds or after drying up of acne. In all of the above cases, consumers rely on cosmetic solutions to their skin appearance problems.

Smooth, soft and glowing skin with even skin tone and colour is thus desired by all consumers who use personal care compositions for their skin. To provide this benefit, manufacturers from around the world have tried many approaches. One very commonly used approach is to include sunscreens or sunblocks in such cosmetic products. Another approach to controlling the colour, tone and appearance of the skin is the so called skin lightening approach where chemicals are added to personal care compositions which alter the formation of melanin in the skin through biochemical transformation in the stratum corneum thereby changing the colour and appearance of the skin. While this approach has been used successfully in many cosmetic products, they often take time for the desired effect to be visible (in the order of weeks or months). Researchers are still struggling to improve the kinetics of skin lightening action.

Consumers, all over the world are desirous of seeing faster and faster effects of the products they use and they expect similar benefits from skin lightening compositions. Thus, there is an unmet demand for providing a composition which produces visibly faster skin lightening as compared to compositions presently available.

A highly suitable base for delivery of such compositions is a cream. Vanishing cream is a widely available and popular base through which skin lightening compositions are delivered. Vanishing cream bases generally comprise 5 to 25% by weight fatty acid. They additionally comprise about 0.1 to 10% by weight soap. Vanishing cream base gives a highly appreciated matty feel to the skin.

The present invention relates to a skin lightening composition that delivers faster skin lightening through such a cream base. The present inventors have found that inclusion of a very selective salt of a dicarboxylic acid viz. disodium fumarate in such a cream base along with selective non-ionic surfactants provides for desired fast skin lightening kinetics without compromising on the desired sensorials afforded by vanishing cream bases.

US 5 002 758 (Kao, 1991) discloses a bathing preparation containing (a) fumaric acid, (b) a carbonate, (c) from 0.1 to 20% by weight, based on fumaric acid, of carboxymethyl cellulose, or an alkali metal salt thereof, having a viscosity of not higher than 2,000 cp in a 1.0% by weight aqueous solution at 25 °C, or at least 0.2% by weight, based on fumaric acid, of polyethylene glycol, and (d) not less than 0.02% by weight and less than 0.1 % by weight, based on fumaric acid, of a nonionic surface active agent having an HLB of 7 or more. The bathing preparation causes neither floating of fumaric acid nor foaming when it is dissolved in a bath nor foaming after it is dissolved and the bath is agitated. US 7 332 152 (Unilever, 2005) discloses a cosmetic composition comprising vitamin B6, vitamin B3 and an organic acid that interacts synergistically to enhance skin lightening.

### Summary of the Invention

According to the present invention there is provided a skin lightening composition comprising,
a) 0.1 % to 5 % by weight disodium fumarate;
b) 0.1 to 5% by weight a non-ionic surfactant selected from the group consisting of
   (i) fatty alcohol ethoxylates with saturated carbon chain and having HLB greater than 16.5, fatty alcohol ethoxylates with unsaturated carbon chain with HLB greater than 12,
   (ii) alkyl phenol ethoxylate, and
   (iii) polyoxyethylene sorbitan mono alkyl esters having carbon chain length 12 to 16 or unsaturated C₁₈ and having an HLB value greater than 12; and
c) a cosmetically acceptable base comprising 1 to 25 % by weight fatty acid.

### Detailed description of the Invention

These and other aspects, features and advantages will become apparent to those of ordinary skill in the art from a reading of the following detailed description and the appended claims. For the avoidance of doubt, any feature of one aspect of the present invention may be utilised in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated. The disclosure of the invention as found herein is to be considered to cover all embodiments as found in the claims as being multiply dependent upon each other irrespective of the fact that claims may be found without multiple dependency or redundancy.

"Skin Lightening Composition" as used herein, is meant to include a composition for topical application to skin of mammals, especially humans. Such a composition may be generally classified as leave-on or rinse off preferably leave-on. It includes any product applied to a human body for getting improved appearance, cleansing, odor control, photoprotection or general aesthetics and is especially useful for providing improved appearance of human skin viz. skin lightening. The composition of the present invention is preferably in the form of a cream and may applied with an implement. Non-limiting examples of personal care compositions include leave-on compositions like skin creams and such compositions may additionally provide benefits of products like antiperspirants, deodorants, depilatories, lipsticks, foundations, mascara, sunscreen lotions, shampoos, or conditioners. "Skin" as used herein is meant to include skin on the face and body (e.g., neck, chest, back, arms, underarms, hands, legs, buttocks and scalp).

The skin lightening composition of the invention comprises a selective salt of dicarboxylic acid (disodium fumarate), a select class of non-ionic surfactants and a cosmetically acceptable base that essentially comprises fatty acid.

The inventors experimented with salts of various other organic acids including monocarboxylic acids, dicarboxylic acid and polycarboxylic acids. From all of these acids they found that disodium fumarate having the structure as shown below 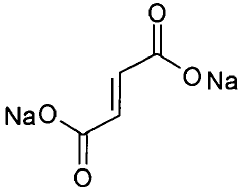 is able to meet the desired fast kinetics of skin lightening. Such a behaviour is not observed with other organic acids that are known to have skin lightening benefits. Disodium fumarate is present in 0.1 to 5%, preferably 0.2 to 3%, further more preferably 0.5 to 2% by weight of the composition.

The composition of the invention comprises non-ionic surfactants of a select class. The non-ionic surfactants suitable for inclusion in the composition of the invention are
(i) fatty alcohol ethoxylates with saturated carbon chain and having HLB greater than 16.5 or
(ii) fatty alcohol ethoxylates with unsaturated carbon chain with HLB greater than 12; or
(iii) alkyl phenol ethoxylate; or
(iv) polyoxyethylene sorbitan mono alkyl esters having carbon chain length 12 to 16 and having an HLB value greater than 12 or
(v) polyoxyethylene sorbitan mono alkyl esters of unsaturated C₁₈ and having an HLB value greater than 12.

Of the above, the classes (i) and (ii) are generally known as the Brij™ surfactants, the class (iii) is generally known as the Triton™ class and the classes (iv) and (v) are called the Tween™ class. 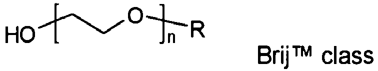 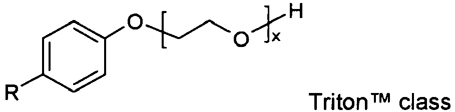 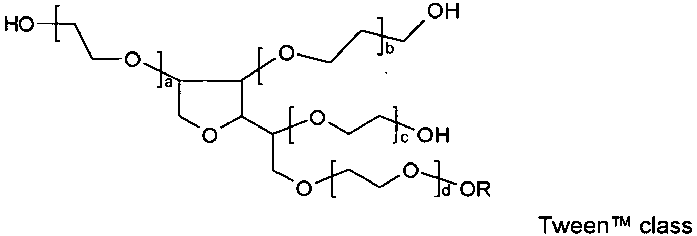 Where R = long carbon chain

While there are very many non-ionic surfactants, only surfactants of the Brij™, Triton™ and Tween™ classes meeting the above criteria are found to meet the desired objects of the invention while anionic, cationic, zwitterionic, amphoteric or non-inonic surfactants not of the above classes or not meeting the above listed criteria are found to not meet the objects of the invention. A list of the non-ionic surfactants suitable for use in the present invention is listed in table 1.

**Table 1**

| Non-ionic surfactant | HLB of non-ionic surfactant | Carbon chain length of non-ionic surfactant | Number of EO groups on non-ionic surfactant |
|---|---|---|---|
| Brij -35 | 17.5 | 12 | 23 |
| Brij -700 | 18.8 | 18 | 100 |
| Brij -97 | 12.4 | 18 (unsaturated) | 10 |
| Brij-99 | 115.3 | 18 (unsaturated) | 20 |
| Triton X 165 | 15.5 | 8 | 16 |
| Triton X 305 | 17.3 | 8 | 30 |
| Triton X 405 | 17.6 | 8 | 35 |
| Triton X 705 | 18.4 | 8 | 55 |
| Tween 80 | 15.2 | 18 (unsaturated) | 20 |
| Tween 20 | 16.6 | 12 | 20 |
| Tween 21 | 13.3 | 12 | 4 |

Without wishing to be bound by theory the present inventors have found that while the select skin lightening agent viz. disodium fumarate is found to have faster acting kinetics of skin lightening, the inclusion of this active in compositions comprising fatty acids, causes problems in the desired sensorials afforded by such compositions and it is through inclusion of the selective class of non-ionics that the kinetics of skin lightening is further improved while restoring the desired skin sensorials. The more preferred non-ionic surfactants are Brij™35, Brij™700, Triton™X405, Tween™20 and Tween™80. The non-ionic surfactant is present in 0.1 to 5%, preferably 0.1 to 3%, further more preferably 0.1 to 2% by weight of the composition.

The composition of the invention comprises a cosmetically acceptable base. The cosmetically acceptable bases are such as to have a product in preferably a cream format. A more preferred format is a vanishing cream base. Vanishing cream base is one which comprises 1 to 25%, preferably 5 to 25%, more preferably 5 to 20% by weight fatty acid. The base preferably comprises 0.1 to 10%, more preferably 0.1 to 3% by weight soap. Preferably the base comprises both fatty acid and soap. C₁₂ to C₂₀ fatty acids are especially preferred in vanishing cream bases, further more preferred being C₁₄ to C₁₈ fatty acids. In creams, the fatty acid is preferably substantially a mixture of stearic acid and palmitic acid. Soaps in the vanishing cream base include alkali metal salt of fatty acids, like sodium or potassium salts The soap is preferably the potassium salt of the fatty acid mixture. The fatty acid in vanishing cream base is often prepared using hysteric acid which is substantially (generally about 90 to 95%) a mixture of 45-47% stearic acid and 53-55% palmitic acid. Thus, inclusion of hysteric acid and its soap to prepare the vanishing cream base is within the scope of the present invention. It is particularly preferred that the composition comprises higher than 7%, preferably higher than 10%, more preferably higher than 12% by weight fatty acid. The cosmetically acceptable base is usually from 10 to 99.9%, preferably from 50 to 99% by weight of the composition. The cosmetically acceptable base preferably includes water. Water is preferably included in 35 to 90%, more preferably 50 to 85%, further more preferably 50 to 80% by weight of the composition. Generally the vanishing cream base in personal care compositions is prepared by taking a desired amount of total fatty matter and mixing with potassium hydroxide in desired amounts. The soap is usually formed in-situ during the mixing.

It is preferred that the composition of the invention has a pH in the range of 6 to 8.

The composition of the invention may additionally comprise another skin lightening agent. The skin lightening agent is preferably chosen from a vitamin B3 compound or its derivative e.g. niacin, nicotinic acid, niacinamide. Other well known skin lightening agents which may be included are aloe extract, ammonium lactate, azelaic acid, kojic acid, butyl hydroxy anisole, butyl hydroxy toluene, citrate esters, 2, 5 dihydroxybenzoic acid and its derivatives, ellagic acid, gluconic acid, glycolic acid, green tea extract, hydroquinone, 4-hydroxy benzoic acid derivatives, hydroxycaprylic acid, lemon extract, linoleic acid, magnesium ascorbyl phosphate, salicylic acid, or vitamins like vitamin B6, vitamin B12, vitamin C, vitamin A, hydroxycarboxylic acid like lactic acid and their salts e.g. sodium lactate, and mixtures thereof. Vitamin B3 compound or its derivative e.g. niacin, nicotinic acid, niacinamide are the more preferred additional skin lightening agent as per the invention, most preferred being niacinamide. Niacinamide, when used, is preferably present in an amount in the range of 0.1 to 10%, more preferably 0.2 to 5% by weight of the composition.

The personal care composition may preferably additionally comprise one or more UV sunscreens. The UV sunscreens may be inorganic or organic.

A safe and effective amount of sunscreen may be used in the compositions of the present invention. The composition preferably comprises from about 0.1% to about 10%, more preferably from about 0.1% to about 5% by weight of a sunscreen agent.

Useful inorganic sun-blocks are also preferably used in the present invention. These include, for example, zinc oxide iron oxide, silica, such as fumed silica, and titanium dioxide.

The composition of the invention may additionally comprise deodorant actives for preparation of a deodorant composition which by way of the invention provides fast kinetics of skin lightening. Deodorant compositions are applied on many areas of the human body but are especially popular for use in the axilla or the underarm area. Deodorant compositions can generally be in the form of firm solids, soft solids, gels, creams, liquids, or sprays and are dispensed using applicators appropriate to the physical characteristics of the composition. For the purposes of the present invention, the composition is preferably in the cream format. Deodorant compositions may or may not additionally comprise antiperspirant actives.

The composition according to the invention may also comprise other diluents. The diluents act as a dispersant or carrier for other materials present in the composition, so as to facilitate their distribution when the composition is applied to the skin.

Diluents other than water can include liquid or solid emollients, solvents, humectants, thickeners and powders.

The cosmetically acceptable base is usually from 10 to 99.9%, preferably from 50 to 99% by weight of the composition, and can, in the absence of other cosmetic adjuncts, form the balance of the composition.

The compositions of the present invention can comprise a wide range of other optional components. The CTFA Cosmetic Ingredient Handbook, Second Edition, 1992, which is incorporated by reference herein in its entirety, describes a wide variety of non-limiting cosmetic and pharmaceutical ingredients commonly used in the skin care industry, which are suitable for use in the compositions of the present invention. Examples include: antioxidants, binders, biological additives, buffering agents, colorants, thickeners, polymers, astringents, fragrance, humectants, opacifying agents, conditioners, exfoliating agents, pH adjusters, preservatives, natural extracts, essential oils, skin sensates, skin soothing agents, and skin healing agents.

The invention is now further described by way of the following non-limiting examples.

### Examples

### Examples 1 to 6: Keratinocyte differentiation of disodium fumarate as compared to other dicarboxylic acids

Several dicarboxylic acids were tested in an in-vitro assay viz. keratinocyte differentiation which is a well known pathway leading to skin lightening. The assay protocol is given below:

5 X 10⁴ HaCaT keratinocyte cells/well were grown in 24 well plates for 24 hours. Cells were cultured with actives for 24 hrs. Cells were then washed with Phosphated buffer solution (PBS) and stained with 5µg/ml acridine orange. Cells were immediately viewed using an epi-fluorescent microscope (Olympus BX 50), at 20X magnification, using appropriate excitation and barrier filters, to simultaneously view red and green fluorescence (excitation: 460 nm and emission > 515 nm). The entire plate was scanned and the percentage of differentiated keratinocytes (cells with red fluorescence evenly distributed within the cytoplasm) was estimated. Data is represented as % control. Increased number suggests increased cell differentiation which is indicative of skin lightening. The data for various actives is presented in table 2.

The experiment in table 2 were carried out at the highest possible concentration above which each of the actives exhibited cytotoxicity.

**Table 2: % Keratinocyte differentiation of various actives**

| Examples | Conc. of active* | Active | average ^{#} | Standard error | t-test |
|---|---|---|---|---|---|
| 1 | 5 mM | sebacic acid | 130 | 2 | 0.03 |
| 2 | 10 mM | disodium fumarate | 183 | 5 | 0.007 |
| 3 | 1 mM | sodium butyrate | 174 | 20 | 0.2 |
| 4 | 10 mM | sodium tartarate | 165 | 6 | 0.028 |
| 5 | 6 mM | sodium succinate | 155 | 35 | 0.1 |
| 6 | 5.5 mM | azeleic acid | 175 | 32 | 0.07 |

| | | | | | |
|---|---|---|---|---|---|
| ^{#} The data presented is an average of minimum three replicates. | | | | | |

### Examples 7 to 10: Melanin synthesis of disodium fumarate as compared to other dicarboxylic acids

B16 melanocytes were cultured in 60 mm² petriplates for 2-3 days or until they reached ∼70% confluency and then actives were added and incubated for 24 hrs. Cells were harvested and protein content was estimated using Bradford method. The cells were then solubilized in 0.1 N NaOH and melanin was estimated. Data are then calculated as mg melanin / mg protein and represented as % control. Decreased numbers indicate inhibition of melanin synthesis by the actives. The data is summarized in table 3.

**Table 3**

| Example | Conc. of active | Active | Average# | Standard error | t-test |
|---|---|---|---|---|---|
| 7 | 15 mM | sebacic acid | 87 | 3 | 0.04 |
| 8 | 10 mM | disodium fumarate | 73 | 2 | 0.05 |
| 9 | 1 mM | sodium butyrate | 92 | 1 | 0.21 |
| 10 | 10 mM | sodium tartarate | 69 | 2 | 0.04 |

| | | | | | |
|---|---|---|---|---|---|
| ^{#} The data presented is an average of minimum three replicates. | | | | | |

The data in tables 2 and 3 indicates that of the various carboxylic acid salts, disodium fumarate is the most promising skin lightening active as evident from data on both keratinocyte differentiation and melanin formation.

### Example 11 and 12: Skin lightening efficacy of compositions with disodium fumarate and a select non-ionic surfactant

Compositions as shown in table 4 were prepared and the skin lightening efficacy was testing using the protocol as described below.

### Short-term Modified Rapid In-Vivo method (MRIV)

This was a double-blind design, in which the study subjects as well as the study personnel applying the products were unaware of the test product composition. Individuals belonging to skin photo type IV and V, a skin colour of 6.5 to 8.0 on a visual numerical scale (VNS) and no marks / scars / blemishes were chosen for the studies, and where the skin phototype is determined according to table 4 below:

**Table 4**

| Type | Also referred to originally as | Tanning behavior | Characteristic hair and eye colour |
|---|---|---|---|
| I | Very light or "Celtic" | Often burns, rarely tans | Tends to have freckles, red, brown or blond hair, blue, brown, or grey eyes |
| II | Light or light-skinned European or "average Caucassion" | Usually burns, sometimes tans | Tends to have light or dark hair, blue, green, hazel, brown or grey eyes |
| III | Light intermediate or dark-skinned European | Rarely bums, usually tans | Usually has brown hair and blue, green, hazel, brown or nearly black eyes |
| IV | Dark intermediate also "Mediterranean" or "Olive skin" | Rarely bums, often tans | Tends to have black to dark brown hair and blue, brown, green or black eyes |
| V | Dark or "Brown" type | Naturally black-brown skin | Black hair and brown or black eyes |
| VI | Very dark or "Black" type | Naturally black-brown skin | Black hair and eyes, with minor variations. |

The main criterion for participation was a history of their response to skin lightening in previous similar type of studies. Both the inner forearms (area from the elbow to wrist), were marked (1 sq cm area) for both treatment and control sites. Application of products was randomized over these sites in order to avoid any positional effects. Color measurements were conducted at baseline (day 0) and after 25 (day 6) and 50 applications (day 11) by trained visual assessor and with Chromameter CM 2500d. A visual numerical scale (VNS) was used by the assessor to score the skin shades. In this scale, a score of 1 was fairest and 10 was darkest. Change in skin color was recorded as 0, -0.125, -0.25, -0.375 etc. Therefore, a score of -0.25 meant the skin color had lightened from, for example, 7.0 to 6.75. Similarly if a formulation caused darkening, a score of +0.25, + 0.50 etc. was given. Skin color was assessed by expert evaluators after 25 (day 6) and 50 (day 11) applications of each formulation. Differences between the treatments after 25 and 50 applications (days 6 and 11) were analyzed using Sigma Stat, SAS and Minitab software. Analysis of variance (ANOVA-normal data) and Kruskal-walli's one way ANOVA on ranks (non-normal data) followed by Tukey test was performed to determine exactly which groups are different. Significance was tested at *p<0.05 and # p<0.10. All data shown represent average skin lightening with standard errors. The skin lightening efficacy of the two compositions is summarized in table 5.

**Table 5**

| Ingredients | Example 11, wt% | Example 12, wt% |
|---|---|---|
| Hysteric Acid | 17.00 | 17.00 |
| Disodium fumarate | 1.00 | 1.00 |
| Non-ionic surfactant (Brij™ 35) | - | 0.25 |
| Glycerine | 1.00 | 1.00 |
| Titanium dioxide | 0.90 | 0.90 |
| Potassium hydroxide | 0.57 | 0.57 |
| Silicone oil | 0.50 | 0.50 |
| Perfume | 0.33 | 0.33 |
| Methyl paraben + propyl paraben | 0.30 | 0.30 |
| Water | To 100 | To 100 |
| Skin lightening score | -0.29 | -0.35 |

The data in table 5 indicates that use of disodium fumarate provides for good skin lightening efficacy while addition of the non-ionic surfactant provides for further enhanced skin lightening.

### Examples 13 to 19: Sensory perception on use of various surfactants

Various compositions similar to example 12 were prepared except that the surfactant was varied as shown in table 6. The sensory perception of the various samples was evaluated by a panel using a protocol as described below.

The samples from example 13 to 19 had been given to a panel of 10 people which had been applied by them on their forearm and rubbed in circular manner from the centre to the circumference until the cream absorbed into the skin. During the process flaky roll-off had been assessed by the panel as "Yes" (for samples having roll-off) and "No" (for samples having no roll-off). A sample with lower number of roll off is preferred.

The data is summarized in table 6.

**Table 6**

| Example | Surfactant | Yes to roll-off | No to roll off |
|---|---|---|---|
| 13 | CTAB | 10 | 0 |
| 14 | Brij ™ 35 | 1 | 9 |
| 15 | Tween™ 20 | 4 | 6 |
| 16 | Tween™ 60 | 8 | 2 |
| 17 | Brij™ 30 | 7 | 3 |
| 18 | Brij™700 | 4 | 6 |
| 19 | Trition™ 405 | 2 | 8 |

| | | | |
|---|---|---|---|
| CTAB: Cetyl trimethyl ammonium bromide Tween™ 60: Polyoxyethylene-20 sorbitan monostearate Brij™ 30: Polyoxyethylene(4) lauryl ether | | | |

The data in table 6 indicates that non-ionic surfactants as per the invention (examples 14, 15, 18 and 19) give good skin sensorials and are liked by consumers while non-ionic surfactants or ionic surfactants outside the invention (examples 13, 16 and 17) give poor skin sensorials and are not liked by consumers.

### Examples 20 and 21: Skin lightening efficacy of composition as per the invention as compared to a conventional skin lightening cream

Compositions as shown in table 7 were prepared and the skin lightening efficacy was tested using the (MRIV) protocol as described earlier.

The skin lightening efficacy of the two compositions at day 6 and day 11 are summarized in table 7.

**Table 7**

| Ingredients | Example 20, wt% | Example 21, wt% |
|---|---|---|
| Hysteric Acid | 17.00 | 17.00 |
| Niacinamide | 1.25 | - |
| Disodium fumarate | - | 1.00 |
| Non-ionic surfactant (Brij™ 35) | - | 0.25 |
| Glycerine | 1.00 | 1.00 |
| Titanium dioxide | 0.90 | 0.90 |
| Potassium hydroxide | 0.57 | 0.57 |
| Silicone oil | 0.50 | 0.50 |
| Perfume | 0.33 | 0.33 |
| Methyl paraben + propyl paraben | 0.30 | 0.30 |
| Water | To 100 | To 100 |
| Skin lightening score (day 6) | -0.24 | -0.31 |
| Skin lightening score (day 11) | -0.26 | -0.39 |

The data in table 7 indicates that a composition as per the invention (example 21) provides for vastly improved skin lightening efficacy as compared to a conventional skin lightening cream.

### Skin lightening efficacy of composition as per the invention as compared to a conventional skin lightening cream in a long term clinical

The same compositions as shown in table 7 were tested using the protocol as described below in a long term clinical.

This was a double-blind study. Both the volar forearms (area from the elbow to wrist), with five sites identified, were selected for treatment. Distribution of products over the various sites was randomized in order to avoid any positional effects. The initial skin color reading on each site served as baseline reading. On the first day of the study (Day 1), subjects who qualified, had two to four test sites (3.0 sq.cm) marked with a template. The skin color was evaluated by an expert assessor using the VNS and by Minolta chromameter CM 2500d. A visual numerical scale (VNS) was used to score the skin shades. In this scale, a score of 1 was fairest and 10 was darkest. Change in skin color was recorded as 0, -0.125, -0.25, -0.375 etc. Therefore, a score of -0.25 meant the skin color had lightened from, for example, 7.0 to 6.75. Similarly if a formulation caused darkening, a score of +0.25, + 0.50 etc. was given. Study personnel applied 9 mg of cream at the designated test site twice daily for 6 weeks. Time between the two applications was three hours (9 am and 12 noon). Change in color of the sites was recorded on weeks 0, 2, 4 and 6. A follow-up was done 40 days after the end of the study to check for skin color. For statistical analysis two measures are used viz. differences between treatments and from baseline. Objective measurements were Sigma Stat, SAS and Minitab software were used for conducting ANOVA for normal data and Freidman's ANOVA, Kruskal - Walli's one way ANOVA on ranks were used for non normal data followed by Tukey test to determine the pair wise significance. The data set was analyzed at *p<0.05 and # p<0.10.

The skin lightening efficacy of the two compositions at week 2, 4 and 6 are summarized in table 8.

**Table 8**

| Time of measurement | Example 20 | Example 21 |
|---|---|---|
| Skin lightening score (End of week 2) | -0.09 | -0.25 |
| Skin lightening score (End of week 4) | -0.23 | -0.37 |
| Skin lightening score (End of week 6) | -0.27 | -0.40 |

The data in table 8 indicates that a composition as per the invention (example 21) provides for better skin lightening and vastly faster kinetics of skin lightening efficacy as compared to a conventional skin lightening cream.

## Claims

1. A skin lightening composition comprising,
a) 0.1 % to 5 % by weight disodium fumarate;
b) 0.1 to 5% by weight a non-ionic surfactant selected from the group consisting of
(i) fatty alcohol ethoxylates with saturated carbon chain and having HLB greater than 16.5, fatty alcohol ethoxylates with unsaturated carbon chain with HLB greater than 12,
(ii) alkyl phenol ethoxylate, and
(iii) polyoxyethylene sorbitan mono alkyl esters having carbon chain length 12 to 16 or unsaturated C₁₈ and having an HLB value greater than 12; and
c) a cosmetically acceptable base comprising 1 to 25 % by weight fatty acid.

2. A composition as claimed in claim 1 wherein the cosmetically acceptable base comprises 0.1 to 10% by weight soap.

3. A composition as claimed in claim 1 or claim 2 wherein said non-ionic surfactant is present in 0.1 to 3% by weight of the composition.

4. A composition as claimed in any one of the preceding claims wherein the cosmetically acceptable base is a cream.

5. A composition as claimed in any one of the preceding claims wherein said fatty acid is hysteric acid.

6. A composition as claimed in any one of claims 2 to 5 wherein said soap is potassium salt of a fatty acid.

7. A composition as claimed in any one of the preceding claims having a pH in the range of 6 to 8.

## Patentansprüche

1. Zusammensetzung zum Aufhellen der Haut,
die Folgendes aufweist:
a) 0,1 bis 5 Gew.-% Dinatriumfumarat;
b) 0,1 bis 5 Gew.-% eines nichtionischen Tensids, das aus der Gruppe ausgewählt ist, bestehend aus:
(i) Fettalkoholethoxylaten mit einer gesättigten Kohlenstoffkette und einem HLB-Wert von mehr als 16,5, Fettalkoholethoxylaten mit einer ungesättigten Kohlenstoffkette mit einem HLB-Wert von mehr als 12,
(ii) Alkylphenolethoxylat und
(iii) Polyoxyethylensorbitanmonoalkylester mit einer Kohlenstoffkettenlänge von 12 bis 16 oder ungesättigten C₁₈ und einem HLB-Wert von mehr als 12; und
c) einen kosmetisch akzeptablen Träger, der 1 bis 25 Gew.-% Fettsäure aufweist.

2. Zusammensetzung nach Anspruch 1,
wobei der kosmetisch akzeptable Träger 0,1 bis 10 Gew.-% Seife aufweist.

3. Zusammensetzung nach Anspruch 1 oder 2,
wobei das nichtionische Tensid mit 0,1 bis 3 Gew.-% der Zusammensetzung vorliegt.

4. Zusammensetzung nach einem der vorstehenden Ansprüche,
wobei der kosmetisch akzeptable Träger eine Creme ist.

5. Zusammensetzung nach einem der vorstehenden Ansprüche,
wobei die Fettsäure Hysterinsäure ist.

6. Zusammensetzung nach einem der Ansprüche 2 bis 5,
wobei die Seife das Kaliumsalz einer Fettsäure ist.

7. Zusammensetzung nach einem der vorstehenden Ansprüche,
die einen pH-Wert im Bereich von 6 bis 8 aufweist.

## Revendications

1. Composition éclaircissant la peau comprenant,
a) de 0,1 % à 5 % en poids de fumarate de disodium ;
b) de 0,1 à 5 % en poids d'un tensioactif non ionique choisi dans le groupe constitué
(i) d'éthoxylates d'alcools gras avec une chaîne carbonée saturée et présentant un HLB supérieur à 16,5, d'éthoxylates d'alcools gras avec une chaîne carbonée insaturée avec un HLB supérieur à 12,
(ii) d'éthoxylate d'alkylphénol, et
(iii) d'esters monoalkyliques de polyoxyéthylène sorbitane présentant une longueur de chaîne carbonée de 12 à 16 ou en C₁₈ insaturée et présentant une valeur HLB supérieure à 12 ; et
c) une base cosmétiquement acceptable comprenant de 1 à 25 % en poids d'acide gras.

2. Composition selon la revendication 1, dans laquelle la base cosmétiquement acceptable comprend de 0,1 à 10 % en poids de savon.

3. Composition selon la revendication 1 ou la revendication 2, dans laquelle ledit tensioactif non ionique est présent dans de 0,1 à 3 % en poids de la composition.

4. Composition selon l'une quelconque des revendications précédentes, dans laquelle la base cosmétiquement acceptable est une crème.

5. Composition selon l'une quelconque des revendications précédentes, dans laquelle ledit acide gras est l'acide hystérique.

6. Composition selon l'une quelconque des revendications 2 à 5, dans laquelle ledit savon est un sel de potassium d'un acide gras.

7. Composition selon l'une quelconque des revendications précédentes présentant un pH dans l'intervalle de 6 à 8.
